# EUROPÄISCHE PATENTSCHRIFT

(11) **EP 1 003 786 B1**
(45) Veröffentlichungstag und Bekanntmachung des Hinweises auf die Patenterteilung: **29.03.2006**
(21) Anmeldenummer: 98940103.9
(22) Anmeldetag: 01.07.1998
(51) Int. Cl.: C07K 14/81, A61K 38/55, A61Q 17/04, A61K 8/64

(54) **PEPTID MIT RADIOPROTEKTIVER WIRKUNG**
PEPTIDE WITH RADIO PROTECTIVE EFFECT
PEPTIDE A EFFET RADIOPROTECTEUR

(30) Priorität: 16.08.1997 DE 19735587
(43) Veröffentlichungstag der Anmeldung: 31.05.2000
(73) Patentinhaber: Eberhard-Karls-Universität Tübingen Universitätsklinikum, 72076 Tübingen (DE)
(72) Erfinder: RODEMANN, Hans, Peter, D-70192 Tübingen (DE); DITTMANN, Klaus, D-72076 Tübingen (DE); GÜVEN, Nuri, D-72076 Tübingen (DE); MAYER, Claus, D-72072 Tübingen (DE)
(74) Vertreter: Otten, Hajo
(86) Internationale Anmeldenummer: PCT/EP1998/004051
(87) Internationale Veröffentlichungsnummer: WO 1999/009065

(56) Entgegenhaltungen:
- WO-A-91/07166
- WO-A-94/09802
- WO-A-94/20121
- S. ANDO ET AL: "Anti-chymotrypsin and anti-elastase activities of a synthetic bicyclic fragment containing a chymotrypsin-reactive site of soybean Bowman-Birk inhibitor" BIOCHIMICA ET BIOPHYSICA ACTA, Bd. 916, 1987, Seiten 527-531, XP002083657
- S. TERADA ET AL: "Studies on the synthesis of proteinase inhibitors" INTERNATIONAL JOURNAL OF PEPTIDE AND PROTEIN RESEARCH, Bd. 15, 1980, Seiten 441-454, XP002083658 COPENHAGEN DK
- M. WAKI ET AL: "synthesis and inhibitory properties of reactive-site peptides of protease inhibitors from peanuts and cucumber" PEPTIDE CHEMISTRY,1987, Seiten 657-662, XP002083659

## Beschreibung

Die vorliegende Erfindung betrifft ein Peptid mit radioprotektiver Wirkung.

Ein derartiges Peptid ist bspw. aus der Publikation von Dittmann, K., et al. (1995): "Bowman-Birk proteinase inhibitor (BBI) modulates radiosensitivity and radiation-induced differentiation of human fibroblasts in culture", Radiotherapy and Oncology 34, Seiten 137-143, bekannt.

Unter einer radioprotektiven Wirkung eines Peptids wird dessen schützende Aktivität für Zellen, Gewebe oder Organismen gegenüber schädigender Strahlung verstanden. Für lebende Organismen besonders schädliche Strahlung ist ionisierende Strahlung sowie UV-Strahlung, also energiereiche Strahlungsarten. Ein Peptid weist dann eine radioprotektive Wirkung auf, wenn die durch Strahlung hervorgerufenen Schädigungen durch dieses Peptid reduziert werden. Die einer radioprotektiven Wirkung zugrundeliegenden Mechanismen sind derzeitig noch völlig ungeklärt.

Durch energiereiche Strahlung hervorgerufene Schäden sind bspw. die Veränderung von DNA, also die Mutagenese, die zu Tumorentstehung führen kann, jedoch auch die Degeneration, Atrophie, Fibrosierung oder Nekrose von Geweben, die hoher Strahlung ausgesetzt sind.

So wird bspw. die Entstehung des malignen Melanoms durch hohe Sonnenbelastung der Haut gefördert.

Der menschliche Organismus ist nicht nur bei hoher Sonnenlichtexposition, sondern auch während der Röntgendiagnostik oder bei einer Strahlentherapie von Tumorerkrankungen mit besonders hohen Strahlungsintensitäten konfrontiert.

Einen Schutz gegen UV-Strahlung bieten z.B. UV-filternde Substanzen wie sie bspw. in Sonnenmilch enthalten sind. Als Schutz gegen ionisierende Strahlung wird die Abschirmung nicht zu bestrahlender Körperteile und die möglichst präzise lokale Anwendung der Strahlung eingesetzt.

In jüngster Zeit wurde erkannt, daß es Peptide gibt, die eine radioprotektive Wirkung entfalten können. Ein derartiges Peptid ist der eingangs erwähnte Bowman-Birk-Protease-Inhibitor (BBI), ein bereits seit langem bekannter Inhibitor der Serin-Proteasen Trypsin und Chymotrypsin, der in großer Menge in der Sojabohne enthalten ist.

Die Aminosäuresequenz des BBI ist bekannt, das entsprechende Gen der Sojabohne wurde bereits 1984 kloniert (Hammond, R.W. (1984): "Molecular Cloning and Analysis of a Gene Coding for the Bowman-Birk Protease Inhibitor in Soybean", J.Biol.Chem. 269, Seiten 9883-9890).

Der BBI umfaßt 71 Aminosäuren und weist ein Molekulargewicht von rund 8.000 Dalton auf. Eines der Charakteristika des BBI ist die Anzahl von vierzehn Cysteinresten, die sieben Disulfidbrücken ausbilden und somit die Faltung oder Sekundärstruktur des BBI wesentlich mitbestimmen.

Durch chemische und enzymatische Spaltung mit Cyanogenbromid (CNBr) und der Protease Pepsin wird der BBI in zwei Hälften gespalten, von denen die eine die Trypsin-inhibierende Aktivität und die andere die Chymotrypsin-inhibierende Aktivität aufweist (Odani, S. and Ikenaka, T. (1978): "Studies on Soybean Trypsin Inhibitors", J. Biochem. 83, Seiten 747-753).

Neben der Funktion als Protease-Inhibitor wurden zwei weitere physiologische Aktivitäten des BBI nachgewiesen, nämlich eine antikarzinogene und die bereits erwähnte radioprotektive Wirkung.

In der Publikation von Clair, B.H.St. (1990) "Suppression of Dimethylhydrazine-induced Carcinogenesis in Mice by Dietary Addition of the Bowman-Birk Protease Inhibitor", Cancer Research 50, Seiten 580-586, wurde gezeigt, daß der BBI eine antikarzinogene Wirkung hat. Bei in vitro-Versuchen mit kultivierten Zellen zeigte sich, daß die Chymotrypsin-inhibierende Domäne eine maligne Transformation der Zellen verhindern konnte. Bei in vivo-Versuchen, bei denen mittels Karzinogenen Tumore bei Mäusen induziert wurden und der BBI oral verabreicht wurde, zeigte sich dagegen, daß die Trypsin-inhibierende Domäne des BBI zur Unterdrückung der Tumorbildung notwendig ist. Diese antikarzinogene Wirkung wird demnach direkt auf die Protease-inhibierende Wirkung des BBI zurückgeführt. Bei den in vivo-Versuchen wurde ferner nachgewiesen, daß autoklavierter, also hitzedenaturierter BBI, keine antikarzinogene Wirkung mehr entfalten konnte.

Die bereits erwähnte radioprotektive Wirkung des BBI wurde in der eingangs genannten Publikation von Dittmann et al. (1995) beschrieben. Hier konnte gezeigt werden, daß der BBI das durch Strahlung hervorgerufene Absterben von kultivierten humanen Fibroblasten reduziert. Fibroblasten sind Bindegewebszellen, die bspw. in großen Mengen in der Haut vorkommen.

In der US 5,376,373 wurde ein Verfahren vorgeschlagen, bei dem der durch Strahlung verursachte Gewichts- und Haarverlust dadurch inhibiert wird, daß ein aus Sojabohnen gewonnenes "Konzentrat", in dem der BBI enthalten ist, oral verabreicht wird. Bei der Isolierung des BBI-Konzentrats wird ein Sojabohnenextrakt mehrfach fragmentiert, präzipitiert, ultrafiltriert, verdünnt und wieder konzentriert, um das radioprotektive Produkt zu erhalten. Welche weiteren Bestandteile, bspw. weitere Protease-Inhibitoren oder ähnliches in dem Konzentrat enthalten sind, ist nicht bekannt.

Ein Problem bei der oralen Aufnahme des BBI mit der Nahrung besteht darin, daß an Ratten nachgewiesen wurde, daß große Mengen von Trypsininhibitoren zu einer Hypertrophie und Hyperplasie von Pankreaszellen sowie zu Körpergewichtsverlust führt. Bei Ratten, die für längere Zeiträume Trypsininhibitoren aus Sojabohnen mit der Nahrung aufnahmen, entwickelten sich sogar Pankreastumore.

Ein weiteres Problem bei der Verabreichung von Serin-Protease-Inhibitoren, vor allem bei einer intravenösen Verabreichung, besteht darin, daß die Blutgerinnung, an der Serin-Proteasen maßgeblich beteiligt sind, gestört werden kann.

Vor diesem Hintergrund ist es Aufgabe der vorliegenden Erfindung, ein Peptid mit radioprotektiver Wirkung bereitzustellen, das mit geringem Aufwand herzustellen ist und bei dem die Nachteile der bereits bekannten radioprotektiven Produkte vermieden werden.

Diese Aufgabe wird erfindungsgemäß dadurch gelöst, daß ein Peptid als radioprotektives Agens bereitgestellt wird, das aus der Sequenz SEQ ID-Nr. 2 oder der Sequenz SEQ ID-Nr. 3 aus dem beiliegenden Sequenzprotokoll besteht.

Daß auch die erfindungsgemäβen fragemente des BBI eine radioprotektive Wirkung aufweisen können, war nicht zu erwarten, da üblicherweise Modifikationen, vor allem wenn sie die Konformation des Peptids betreffen, die physiologischen Aktivitäten eines Peptids zerstören oder zumindest stark reduzieren.

Darüber hinaus war für die antikarzinogene Aktivität des BBI gezeigt worden, daß durch Beeinträchtigung der BBI-Struktur durch Hitzedenaturierung dessen antikarzinogene Wirkung völlig verloren ging.

Die erfindungsgemäßen Fragmente des BBI sind problemlos zu handhaben, weil sowohl während deren Herstellung als auch Aufbewahrung nicht darauf geachtet werden muß, daß ein Kontakt mit modifizierenden Agenzien, Proteasen oder ähnlichem ausgeschlossen ist.

Somit wird die der Erfindung zugrundeliegende Aufgabe vollkommen gelöst.

Das Peptid weist keine Protease-inhibierende Wirkung gegen Trypsin und Chymotrypsin auf.

Ein derartiges Peptid hat den erheblichen Vorteil, neben der radioprotektiven Wirkung nicht gleichzeitig zusätzlich noch die Proteasen Trypsin oder Chymotrypsin zu blockieren. Dies ist besonders dann vorteilhaft, wenn das Peptid zum Schutz von Zellen, Geweben oder Organismen gegen Strahlung eingesetzt werden soll, denn, wie oben bereits erläutert wurde, sind zumindest Peptide mit Trypsin-inhibierender Wirkung schädlich für Pankreaszellen und können sogar zur Tumorbildung führen.

Ein erfindungsgemäßes Peptid hat eine einzige wohl-definierte Wirkung, nämlich dem Schutz vor Strahlung. Wenn es in Anwesenheit von Trypsin und Chymotrypsin eingesetzt wird, werden diese Serin-Proteasen nicht gleichzeitig in ihrer Aktivität behindert. So werden auch die oben aufgeführten Pankreas-schädigenden Nebenwirkungen vermieden.

Die zwei ggf. in reduzierter Form vorliegenden Cysteinreste des einen erfindungsgemäßen Peptides bringen den Vorteil mit sich, daß ein derartiges Peptid auch in Anwesenheit von reduzierenden Agenzien als radioprotektives Agens eingesetzt werden kann. Reduzierende Agenzien führen bei Peptiden, die Cysteinreste enthalten, dazu, daß ihre Schwefelreste protoniert werden und damit als SH-Gruppen vorliegen. Unter oxidierenden Bedingungen bilden die SH-Gruppen untereinander Disulfidbrücken aus, die die Faltung des Peptids wesentlich mitbestimmen.

Reduzierte Agenzien werden häufig bei der Reinigung von Proteinen und Peptiden eingesetzt. Unter physiologischen Bedingungen liegen im Inneren von Zellen aufgrund der Anwesenheit von Glutathion reduzierende Bedingungen vor.

Die Bereitstellung eines Peptids, dessen Cysteinreste in reduzierter Form vorliegen, hat den weiteren Vorteil, daß es problemlos mit molekularbiologischen Methoden in Bakterien hergestellt werden kann. Eine solche Expression in Bakterien ist gegenüber der Expression in Hefen und höheren eukaryontischen Zellen wesentlich einfacher und effizienter und erlaubt besonders hohe Ausbeuten. Für Proteine oder Peptide, bei denen die Cysteinreste über Disulfidbrücken kovalent miteinander verbunden sind, ist dieses System jedoch ungeeignet, da in Bakterien keine Disulfidbrücken ausgebildet werden können. Eine reduzierte Form des BBI kann dagegen ohne weiteres in Bakterien exprimiert werden.

In einer weiteren bevorzugten Ausgestaltung liegen zumindest einige der Aminosäurereste des Peptids in alkylierter Form vor.

Unter Alkylierung versteht man die Modifikation einzelner Aminosäuren mit Alkylgruppen, meist Methylgruppen. Diese Modifikation erfolgt mit sogenannten Alkylierungsreagenzien, bspw. mit Jodacetamid.

Durch die Behandlung des BBI mit Jodacetamid nach Reduktion der Disulfidbrücken werden die SH-Gruppen der Cysteine mit einer Methylgruppe versehen, also alkyliert. Eine Reoxidation der SH-Gruppen ist dann nicht mehr möglich. So wird selbst unter oxidierenden Bedingungen verhindert, daß sich die Disulfidbrücken wieder ausbilden.

Eine Alkylierung des BBI oder von Fragmenten des BBI hat den Vorteil, die Cysteinreste in ihrer reduzierten Form zu erhalten und damit zu stabilisieren.

Das erfindungsgemäße Peptid weist sieben oder neun Aminosäuren auf.

Ein solches drastisch verkleinertes BBI-Fragment hat den erheblichen Vorteil, sich bei seinem Einsatz auf Zellen, Geweben oder im ganzen Organismus aufgrund der geringen Größe wesentlich besser und schneller zu verteilen und einzudringen als der über 70 Aminosäuren umfassende Gesamt-BBI.

Eine gute Verteilung sowie ein leichtes Eindringen in das Gewebe ist bei der Verwendung des Peptids als Radioprotektor wesentlich, da es dann schnell und umfassend seine schützende Wirkung gegen Strahlung entfalten kann.

Ein weiterer Gegenstand der vorliegenden Erfindung betrifft die Verwendung eines Nonapeptides, das die Sequenz SEQ ID-Nr.: 1 aus dem anliegenden Sequenzprotokoll aufweist und in linearer Form vorliegt, als radioprotektives Agens.

Wie den Ausführungsbeispielen zu entnehmen ist, ist es den Erfindern gelungen, nachzuweisen, daß dieses Nonapeptid eine ebenso hohe radioprotektive Wirkung auf menschliche Fibroblasten ausübt wie der Gesamt-BBI. Der Einsatz eines gegenüber dem Gesamt-BBI sehr stark verkleinerten Peptids, das nur neun Aminosäuren umfaßt (Nonapeptid), hat den wesentlichen Vorteil, daß dieses Nonapeptid leichter hergestellt werden kann. Ein Nonapeptid kann nämlich problemlos durch chemische Synthese, auch Merrifield-Synthese genannt, produziert werden. Diese Art der Peptidsynthese ist eine weit verbreitete und gut etablierte Synthesemethode, durch die ein derartiges Peptid in großen Mengen und in hoher Reinheit gewonnen werden kann.

Besonders überraschend waren die radioprotektiven Eigenschaften dieses Nonapeptids auch deshalb, da es sich hierbei um ein solches Peptid handelt, das im Stand der Technik grundsätzlich bekannt war. So beschreiben Terada et al. (1980), "studies on the synthesis of proteinase inhibitors", Int. J. Peptide Res. 15, Seiten 441 bis 454, ein Peptid, das die Sequenz SEQ ID-Nr. 1 aufweist. Die Autoren erkennen jedoch nicht, daß dieses Peptid eine radioprotektive Wirkung zeigt.

Bei dem eingangs genannten erfindungsgemäßen Peptid mit radioprotektiver Wirkung, das die Sequenz SEQ ID-Nr.: 2 aus dem anliegenden Sequenzprotokoll aufweist, wurde die aus dem natürlich vorkommenden BBI entnommene Sequenz um eine Aminosäure abgewandelt. Das Serin aus der Sequenz SEQ ID-Nr.: 1, also eine hydrophile Aminosäure, wurde in der SEQ ID-Nr.: 2 gegen ein Valin, also eine hydrophobe Aminosäure ausgetauscht. Auch für dieses modifizierte Peptid konnte eine radioprotektive Wirkung nachgewiesen werden, wie aus den Ausführungsbeispielen ersichtlich ist. Auch dieses modifizierte Nonapeptid mit der Sequenz SEQ ID-Nr. : 2 kann problemlos mittels Merrifield-Synthese synthetisiert werden.

In einer bevorzugten Ausführung sind die terminalen Cysteinreste des Peptides mit der Sequenz SEQ ID-Nr.: 2 kovalent miteinander verbunden.

Auf diese Weise entsteht ein ringförmiges oder zyklisches Peptid, das in einem oxidierenden Milieu eine hohe Stabilität aufweist. Ein oxidierendes Milieu liegt bspw. in der extrazellulären Matrix des Bindegewebes sowie an allen Körperteilen vor, die Kontakt mit der Außenluft haben.

Da sowohl die linearen als auch die zyklischen Peptide mit der Sequenz SEQ ID-Nr. 2 und das lineare Peptid mit der Sequenz SEQ ID NO:1 eine radioprotektive Wirkung aufweisen, sind diese sowohl in reduzierender als auch in oxidierender Umgebung als Strahlenschutz wirksam und somit universell einsetzbar.

Das eingangs genannte erfindungsgemäße Peptid mit radioprotektiver Wirkung, das die Sequenz SEQ ID-Nr.: 3 aus dem anliegenden Sequenzprotokoll aufweist, umfaßt nur sieben Aminosäuren, es ist also ein Heptapeptid. Durch die weitere Verkürzung des Peptids unter Erhalt seiner radioprotektiven Wirkung ist das Peptid noch schneller und preiswerter herzustellen sowie leichter zu applizieren.

Alle Peptide mit den Sequenzen SEQ ID-Nr.: 1 - 3 haben den erheblichen Vorteil, keine Protease-inhibierende Aktivität zu haben. Sie können somit ohne die unerwünschte Nebenwirkung der Blockierung der Verdauungs - enzyme Trypsin und Chymotrypsin und anderer Serin-Proteasen eingesetzt werden.

In einer weiteren bevorzugten Ausgestaltung weist zumindest eine der Aminosäuren des Peptids eine Schutzgruppe auf.

Derartige Schutzgruppen können beliebige der in der Peptidchemie bekannten Schutzgruppen sein, wobei es bevorzugt ist, daß die C-terminale Aminosäure eine Acetylgruppe und/oder die N-terminale Aminosäure eine Amidgruppe aufweist.

Diese Schutzgruppen haben den Vorteil, die Peptide vor dem Angriff von Exopeptidasen zu schützen, so daß die Peptide in einem biologischen Milieu wie bspw. in der Zellkultur oder im Organismus eine wesentlich höhere Stabilität haben. Schutzgruppen, die die C-terminale Carboxyl- und die N-terminale Aminogruppe von Peptiden blockieren, wie die genannten Acetyl- bzw. Amidgruppen, sorgen ferner dafür, daß die Peptide keine weiteren Peptidbindungen mit anderen Peptiden oder untereinander eingehen, so daß auch Tandemisierungen der Peptide sicher verhindert werden. Bei mehreren untereinander verknüpften Peptiden kann nicht mehr sicher davon ausgegangen werden, daß die radioprotektive Wirkung erhalten ist.

Demnach sorgen die Schutzgruppen auch dafür, daß die Peptide in ihrer radioprotektiven wirksamen Struktur erhalten bleiben.

Ein weiterer Aspekt der vorliegenden Erfindung ist die Verwendung eines oder mehrerer der genannten Peptide zur Herstellung eines radioprotektives Mittels.

Diese Verwendung umfaßt jede Verwendung, bei der die Peptide zum Schutz gegen Strahlung, sei es ionisierende Strahlung, UV-Strahlung oder elektromagnetische Strahlung, eingesetzt werden.

Besonders bevorzugt wird ein erfindungsgemäßes Peptid zum Schutz gegen ionisierende Strahlung, insbesondere von Normalgewebe bei der Strahlentherapie von Tumorpatienten, eingesetzt.

Bei einer derartigen Strahlentherapie werden nämlich ionisierende Strahlungen zur Behandlung meist maligner Tumoren verwendet. Dabei soll das Tumorgewebe maximal geschädigt werden und gleichzeitig das umgebende gesunde Normalgewebe maximal geschont werden.

Um die Belastung des Normalgewebes möglichst gering zu halten, wird die Strahlung wenn möglich lokalisiert eingesetzt. Sehr problematisch ist jedoch der Schutz des in der direkten Umgebung des Tumorgewebes liegenden Normalgewebes. Hier kann ein erfindungsgemäßes Peptid ideal eingesetzt werden, da es entweder lokal oder, z.B. über die Blutbahn, generalisiert eingesetzt werden kann. Insbesondere bei den kleinen Peptiden mit den Sequenzen SEQ ID-Nr.: 1 - 3 wird eine schnelle und homogene Verteilung in den Geweben erreicht, und somit auch eine schnelle schützende Wirksamkeit gegenüber der Strahlung. Zudem weisen alle erfindungsgemäßen Peptide sowohl in oxidierender als auch in reduzierender Umgebung eine hohe Stabilität und zugleich radioprotektive Wirksamkeit auf, was ihre therapeutische Verwendbarkeit zusätzlich begünstigt.

Bevorzugt wird ein erfindungsgemäßes Peptid auch zur Herstellung eines Mittels zum Schutz der Haut gegen UV-Strahlung, insbesondere gegen die UV-Strahlung des Sonnenlichts eingesetzt.

Hierbei ist vorteilhaft, daß die Peptide auch unter oxidierenden Bedingungen, also bspw. an der Luft, sehr beständig sind und eine lang anhaltende Schutzwirkung gegen W-Strahlen ausüben können. Sie sind damit geeignet, als Hautschutz gegen hohe Sonneneinstrahlungen eingesetzt zu werden. Der Einsatz der erfindungsgemäßen Nona- und Heptapeptide hat ferner den Vorteil, daß die Peptide wegen ihrer geringen Größe in die Haut eindringen können und dort lange beständig sind.

Die Erfindung betrifft außerdem eine pharmazeutische Zusammensetzung, insbesondere zur intravenösen Verabreichung, die eines oder mehrere der erfindungsgemäßen Peptide in einer radioprotektiven wirksamen Menge enthält.

Dazu können die Peptide in den jeweils angemessenen und üblichen Galeniken zubereitet sein. Neben einer intravenösen Verabreichung kann auch eine perkutane Verabreichung oder eine lokale Injektion in bspw. durch Bestrahlung direkt betroffene Körpergebiete oder Körperhöhlen in Betracht gezogen werden.

Bei einer derartigen pharmazeutischen Zusammensetzung ist vorteilhaft, daß die Peptide ihre radioprotektive Wirkung entfalten, ohne gleichzeitig bedrohliche Immunreaktionen auszulösen. Aufgrund ihrer geringen Größe haben die erfindungsgemäßen Peptide nämlich nur eine geringe Immunogenität, so daß unter normalen Umständen bei Verwendung der pharmazeutischen Zusammensetzung am menschlichen oder tierischen Körper keine allergischen Reaktionen zu erwarten sind, und die Peptide auch nicht unter Vermittlung von Antikörpern aus dem jeweiligen Organismus eliminiert werden.

Die Erfindung betrifft auch eine kosmetische Zusammensetzung zum Auftragen auf die Haut, die dadurch gekennzeichnet ist, daß sie eines oder mehrere der erfindungsgemäßen Peptide in einer radioprotektiv wirksamen Menge enthält.

Eine derartige kosmetische Zusammensetzung kann bspw. als Sonnenmilch, Hautcreme oder ähnliches dargereicht werden und enthält dann die üblichen Bestandteile derartiger Zusammensetzungen wie Öle, Emulsionen, Pigmente usw. Es versteht sich, daß die kosmetische Zusammensetzung zusätzlich auch UV-Filter wie Derivate von p-Aminobenzoesäure, Salizylsäure, Zimtsäure, Dibenzoylmethan oder ähnliches enthalten kann.

Durch die radioprotektive Wirksamkeit der erfindungsgemäßen Peptide bietet eine solche kosmetische Zusammensetzung einen idealen Schutz vor allem gegen die UV-Strahlung des Sonnenlichts. Da die erfindungsgemäßen Peptide aufgrund ihrer geringen Größe sogar in die Haut eindringen und zudem lange beständig sind, kann so ein Langzeitschutz gegen Strahlung erreicht werden.

Die Erfindung betrifft ferner eine Nukleinsäure, die aus einem für ein erfindungsgemäßes Peptid codierenden Sequenzabschnitt sowie ggf. zur Expression der Nukleinsäure notwendige Kontrollsequenzen besteht.

Eine derartige Nukleinsäure wird vorteilhafterweise zur Herstellung eines erfindungsgemäßen Peptids mit molekularbiologischen Techniken verwendet, wozu sie bevorzugt in einem Expressionsvektor enthalten ist.

Die Herstellung eines erfindungsgemäßen Peptids durch Nukleinsäureexpression hat den Vorteil, eine besonders einfache Möglichkeit zu sein, das Peptid in praktisch unbegrenzten Mengen herzustellen und auf einfache Art und Weise abzuwandeln, indem die entsprechende codierende Sequenz auf Nukleinsäureebene modifiziert wird. Hierfür sind eine Reihe von Standardverfahren wie in vitro-Mutagenese, site-directed mutagenesis, Oligonukleotidsynthese, PCR usw. bekannt.

Als Expressionssystem kann entweder ein in vitro-Expressionssystem, bspw. ein Retikulozyten-Lysat, oder die in vivo-Expression in Bakterien, Hefen oder eukaryontischen Zellen eingesetzt werden, wobei jeweils geeignete Expressionsvektoren verwendet werden. Da zur radioprotektiven Wirkung der erfindungsgemäßen Peptide die Ausbildung von Disulfidbrücken nicht unbedingt notwendig ist, kann die Expression in Bakterien, in denen Disulfidbrücken nicht ausgebildet werden, erfolgen.

Zur Erleichterung der Herstellung und Reinigung der erfindungsgemäßen Peptide können diese auch als Fusionspeptide synthetisiert werden, was bedeutet, daß für Aminosäureabschnitte oder Domänen bekannter Proteine codierende Sequenzen an die erfindungsgemäßen Nukleinsäuren anfusioniert werden, wodurch bei der Expression dann ein durchgehendes Peptid erzeugt wird. Beispiele für solche anfusionierten Aminosäureabschnitte sind bspw. sogenannte Histidin-Tags, durch die exprimierte Fusionsproteine über Nickel-Chelat-Säulen gereinigt werden können, oder antigene Determinanten, die es erlauben, die Peptide über geeignete Antikörperaffinitätssäulen zu reinigen.

Bei einem alternativen bevorzugten Verfahren zur Herstellung eines erfindungsgemäßen Peptids wird der native BBI proteolytisch und/oder chemisch gespalten.

Bei diesem Verfahren kann von einem aus Sojabohnen(samen) gereinigten BBI oder molekularbiologisch hergestellten BBI ausgegangen werden, und die radioprotektiv wirksamen modifizierten Formen oder Fragmente des BBI werden daraus dann mit (bio)-chemischen Methoden gewonnen. Dabei können z.B. Proteasen verwendet werden, die den BBI spalten, bspw. Pepsin, und/oder chemische Spaltungen durch cyanogenbromid erfolgen.

Weitere Vorteile ergeben sich aus den folgenden Ausführungsbeispielen und im Zusammenhang mit der Zeichnung, in der:
- Fig. 1: das Ergebnis eines sogenannten klonogenen Assays mit nativem und modifiziertem BBI in Form eines Balkendiagramms zeigt;
- Fig. 2: als Balkendiagramm die Chymotrypsin-inhibierende Aktivität von nativem und modifiziertem BBI zeigt;
- Fig. 3: das Elutionsprofil einer chromatographischen Auftrennung von BBI und BBI-Fragmenten zeigt;
- Fig. 4: als Balkendiagramm das Ergebnis eines klonogenen Assays mit ausgewählten Fraktionen der Chromatographie aus Fig. 3 zeigt;
- Fig. 5: als Balkendiagramm das Ergebnis eines klonogenen Assays mit BBI und vier erfindungsgemäßen Peptiden unter ionisierender Strahlung zeigt;
- Fig. 6: als Balkendiagramm das Ergebnis eines klonogenen Assays mit BBI und vier erfindungsgemäßen Peptiden unter UV-Bestrahlung zeigt;
- Fig. 7: als Balkendiagramm die Chymotrypsin-inhibierende Aktivität von BBI und vier erfindungsgemäßen Peptiden zeigt; und
- Fig. 8: als Balkendiagramm die Trypsin-inhibierende Aktivität von BBI und vier erfindungsgemäßen Peptiden zeigt.

### Beispiel 1 Radioprotektive Wirkung sowie Protease-inhibierende Aktivität von BBI und modifizierten BBI-Formen (Vergleichsbeispiel)

### A Radioprotektive Wirkung

Die Untersuchung der radioprotektiven Wirkung des BBI sowie modifizierter Formen des BBI wurde im sogenannten klonogenen Assay durchgeführt, der bspw. bei Dittmann, K. et al., Radiotherapy and Oncology 34 (1995), Seiten 137-143, beschrieben ist und der im folgenden kurz erläutert wird.

### 1.1 Klonogener Assay

Normale humane Fibroblasten wurden in Dulbecco's Modified Eagles Medium (DMEM) mit 10 % fötalem Kälberserum unter Standardbedingungen kultiviert. Bei jeder Zellpassage wurde die Zellzahl bestimmt und die Zellen beim Anlegen von Subkulturen mit einer Dichte von 2 x 10⁴ pro cm² ausgesät.

Für den klonogenen Assay wurden sekundäre Fibroblasten mit 0,05 % Trypsin und 0,1 % EDTA trypsiniert und mit einer Zelldichte von 50 Zellen pro cm² in 6-Well-Gewebekulturschalen ausgesät. Die Zellen wurden mit 2 ml DMEM mit 20 % fötalem Kälberserum je Vertiefung (Well) kultiviert.

Nach 24 Stunden wurde das Medium entfernt und die Zellen entweder in zusatzfreiem Kontrollmedium (in den Figuren: "O") oder in Medium, das jeweils 10 *µ*M des BBI oder einer modifizierten BBI-Form oder eines BBI-Fragmentes enthielt, für 16 Stunden inkubiert. Die erfindungsgemäßen Nona- und Heptapeptide (siehe Beispiel 3) wurden mit einer Konzentration von 80 µM eingesetzt.

Danach erfolgte die Bestrahlung mit ionisierender Strahlung, wobei die Energiedosis entweder 2 oder 4 Gray (Gy) betrug. Diese Energiedosis entspricht der gesamten umgesetzten Strahlungsenergie in der Masseneinheit Joule/kg = (Gray). Zur Bestrahlung wurde ein 6 MeV-Linearbeschleuniger (Mevatron, Firma Siemens) verwendet.

Danach wurden die Zellen in BBI-freiem Kulturmedium für acht Tage weiter kultiviert, um eine Koloniebildung zu erlauben.

Dann wurden die Zellen fixiert, gefärbt und ausgezählt, wobei in den Figuren "K" die absolute Anzahl an ausgezählten Klonen angibt.

Ein Klon ist dabei eine Kolonie oder ein Zellhaufen, der durch Teilung einer Zelle innerhalb der 8-tägigen Kultivierung entstanden ist. Die Anzahl an Klonen entspricht dem Ausmaß, in dem die menschlichen Fibroblasten die Bestrahlung überlebt haben. Es wird daher im folgenden auch von dem "klonogenen Überleben" der Zellen gesprochen. Wenn bei der Bestrahlung der Zellen viele Zellen sterben, so bilden sich nach acht Tagen nur wenige Klone aus, überleben viele Zellen, so lassen sich nach 8-tägiger Kultivierung viele Klone auszählen. Somit ist das klonogene Überleben der Zellen nach Bestrahlung ein direktes Maß für die radioprotektive Wirkung der eingesetzten BBI-Produkte.

### 1.2 Radioprotektive Wirkung von BBI und modifizierten BBI-Formen

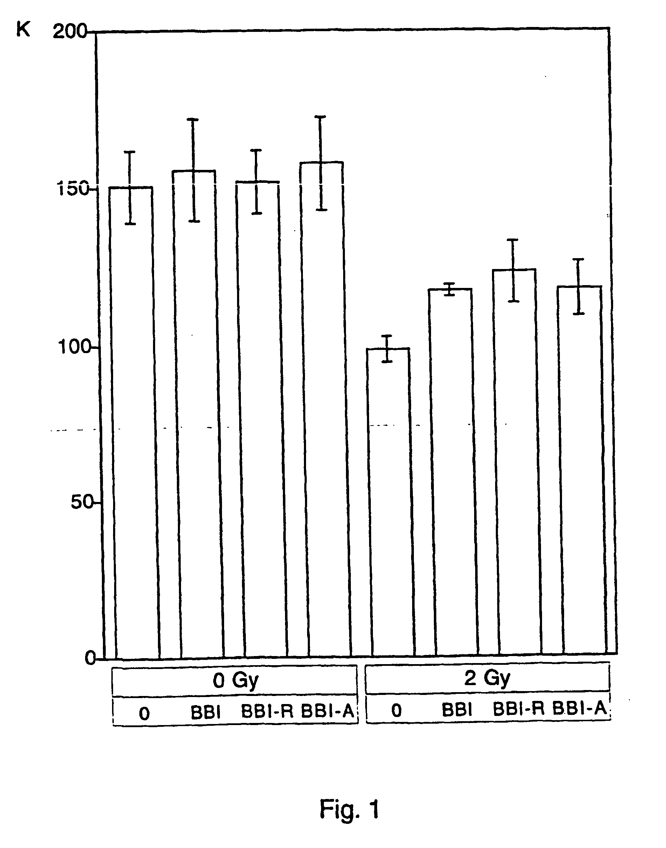

Bei dem klonogenen Assay, dessen Ergebnis in Fig. 1 gezeigt ist, wurden vier verschiedene Ansätze getestet: Bei dem mit "0" bezeichneten Kontrollansatz wurde kein BBI eingesetzt. Bei dem mit "BBI" bezeichneten Ansatz wurde Gesamt-BBI (von der Firma Sigma Biochemicals) eingesetzt, bei dem mit "BBI-R" bezeichneten Ansatz war der Gesamt-BBI mit dem reduzierenden Agens Dithiothreitol (DTT) vorbehandelt worden, so daß alle Disulfidbrücken des BBI aufgespalten waren.

Bei dem mit "BBI-A" bezeichneten Ansatz wurde der BBI zunächst reduziert und die Cysteinreste dann mit Jodacetamid alkyliert, um eine Reoxidation der Cysteinreste zu vermeiden. Zu dieser Reduktion wurden 8 mg BBI in 0,5 ml PBS und 0,2 ml Denaturierungspuffer (12,5 mM Tris pH 6,8, 80 *µ*l EDTA, 1% SDS und 20 % Glycerin) in 20 *µ*l frischer 2,6 M DTT-Lösung für 10 min gekocht. Die Alkylierung erfolgte nach der Reduktion durch Zusatz von 70 *µ*l 20%iger Jodacetamid-Lösung für 60 Minuten bei 20°C. Danach wurden die Ansätze viermal gegen PBS für 24 Stunden dialysiert.

Die vier Ansätze 0, BBI, BBI-R und BBI-A wurden entweder nicht mit ionisierender Strahlung behandelt (0 Gy) oder mit einer Einzeldosis von 2 Gy bestrahlt.

Dieser Test wurde in mehreren unabhängigen Experimenten wiederholt und die jeweiligen Ergebnisse gemittelt. Die Fehlerbalken geben die Streubreite der Werte an.

Wie Fig. 1 zu entnehmen ist, beeinflußt der Zusatz von BBI, reduziertem BBI oder reduziertem und alkyliertem BBI ohne Bestrahlung (O Gy) das Überleben der Zellen im Rahmen der Meßgenauigkeit nicht. Ohne Zusatz von BBI (O) wird das klonogene Überleben von menschlichen Hautfibroblasten nach Bestrahlung mit einer Einzeldosis von 2 Gy um ca. 30 bis 40 % reduziert.

Eine 16-stündige Vorbehandlung mit BBI (BBI), reduziertem BBI (BBI-R) oder reduziertem und alkyliertem BBI (BBI-A) führte zu einer signifikanten Steigerung des klonogenen Überlebens der bestrahlten menschlichen Hautfibroblasten um 20 - 30 %.

Signifikante Unterschiede zwischen BBI, BBI-R und BBI-A waren im Rahmen der Meßgenauigkeit nicht nachweisbar.

Das in Fig. 1 gezeigte Ergebnis des klonogenen Assays belegt, daß modifizierte Formen des BBI, nämlich dessen reduzierte Form oder reduzierte und alkylierte Form, eine ebenso hohe radioprotektive Wirkung wie der unveränderte BBI aufweisen.

### B Inhibitor-Wirkung

Als nächsts wurde untersucht, ob die modifizierten Formen des BBI als Protease-Inhibitoren gegenüber Chymotrypsin wirken oder nicht. Dazu wurde ein Protease-Inhibitionstest durchgeführt, der im folgenden kurz erläutert wird.

### 1.3 Protease-Inhibitionstest

Ein Ansatz mit 50 *µ*l TLCK-behandeltem Chymotrypsin (0,1 mg/ml) wurde mit 50 *µ*l einer Lösung mit BBI (BBI), reduziertem BBI (BBI-R) oder reduziertem und alkyliertem BBI (BBI-A) zusammen mit 50 *µ*l PBS und 50 *µ*l des Chymotrypsin-Substrats Acetyl-A-A-P-F-pNa (0,5 mg/ml von Bachem Heidelberg, BRD) in 12,5 % DMSO, 87,5 % PBS für 10 Minuten inkubiert. Das farbige Reaktionsprodukt wird bei 405 nm in einem Spektrophotometer nachgewiesen.

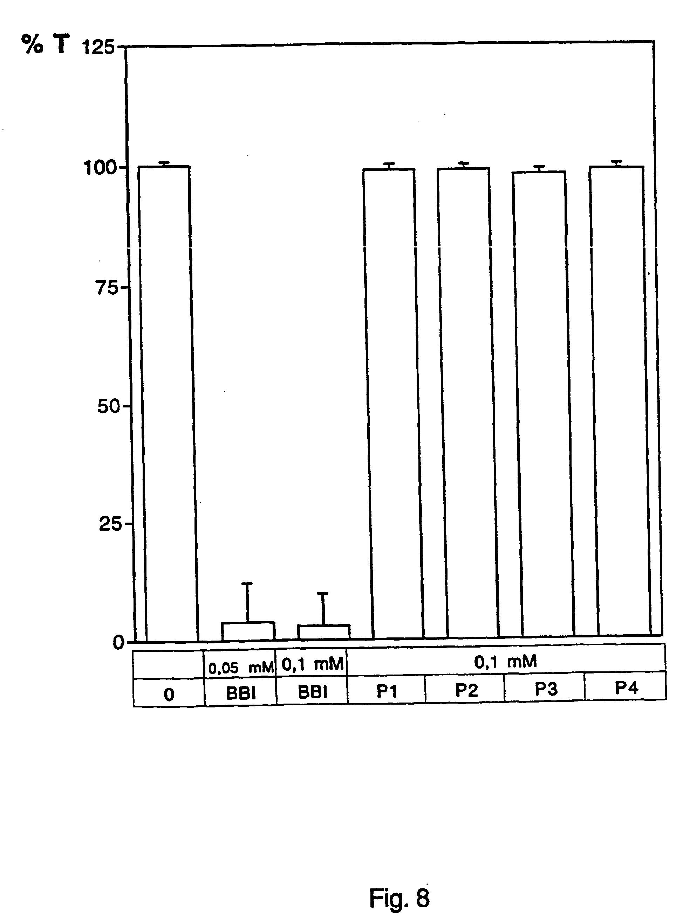

Sollte die Inhibition der Protease Trypsin (siehe Beispiel 4, Fig. 8) bestimmt werden, so wurde als Substrat das Peptid CBZ-R-pNA (1 mg/ml) sowie TPCK-behandeltes Trypsin (0,1 mg/ml) verwendet und der Test im übrigen wie beschrieben durchgeführt.

Als Kontrolle wurde ein Ansatz ohne BBI (O) durchgeführt, dessen Meßwert mit 100 % Chymotrypsinaktivität (% CH) gleichgesetzt wurde. Der Blankwert mit Puffer allein ist mit "-" bezeichnet und gibt den Hintergrund der Pufferlösung und der Küvette an.

### 1.4 Inhibition von Chymotrypsin durch BBI und modifzierte BBI-Formen

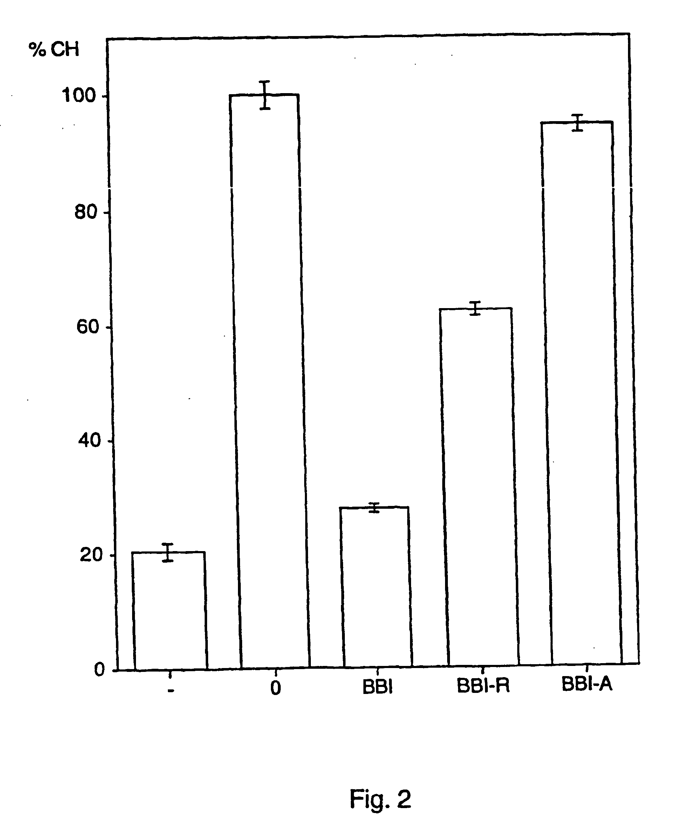

Das Ergebnis des Chymotrypsin-Tests ist in Fig. 2 gezeigt. Während der Gesamt-BBI die Chymotrypsinaktivität um 80 % inhibiert, kann die reduzierte Form des BBI (BBI-R) die Chymotrypsinaktivität nur um ca. 30 % inhibieren. Dagegen ist die reduzierte und alkylierte Form des BBI (BBI-A) nicht mehr dazu in der Lage, die Chymotrypsinaktivität zu inhibieren.

Die bei einem Einsatz der modifizierten Formen des BBI (BBI-R und BBI-A) als radioprotektive Agenzien unerwünschte Nebenwirkung, daß Chymotrypsin, ein Verdauungsenzym, gleichzeitig inhibiert wird, ist somit bei den modifizierten BBI-Formen stark reduziert oder fehlt völlig.

### Beispiel 2 Spaltung des BBI mit Cyanogenbromid und Pepsin sowie Analyse der Spaltprodukte auf Protease-Inhibition und radioprotektive Aktivität (Vergleichsbeispeil)

In diesem Experiment wurde nachgewiesen, daß auch Teilfragmente des BBI eine radioprotektive Wirkung aufweisen.

### 2.1 Spaltung des BBI.

Der Gesamt-BBI wurde mit Cynaogenbromid (CNBr) und mit dem Magenenzym Pepsin verdaut. Dazu wurden 50 mg BBI in 1,5 ml 70%iger Ameisensäure verdünnt. Dann wurden 118 mg Cyanogenbromid zugegeben und der Ansatz 20 Stunden bei 4°C inkubiert. Das Reaktionsgemisch wurde mit Wasser verdünnt und lyphilisiert. Das Lyophilisat wurde dann mit 340 U Pepsin bei pH 2,5 für 24 Stunden bei 40°C verdaut. Um die Verdauungsreaktion zu beenden, wurde Ameisensäure hinzugegeben.

### 2.2 Auftrennung der Spaltungsprodukte

Das gespaltene Material wurde dann durch Molekularsieb-Chromatographie auf einer Sephadex G25-Säule aufgetrennt. Während des Säulenlaufs wurden 110 Fraktionen gewonnen, deren inhibitorische Aktivität gegenüber Trypsin oder Chymotrypsin in dem unter 1.3 aufgeführten Protease-Inhibitionstest untersucht wurde.

### 2.3 Protease-Inhibition

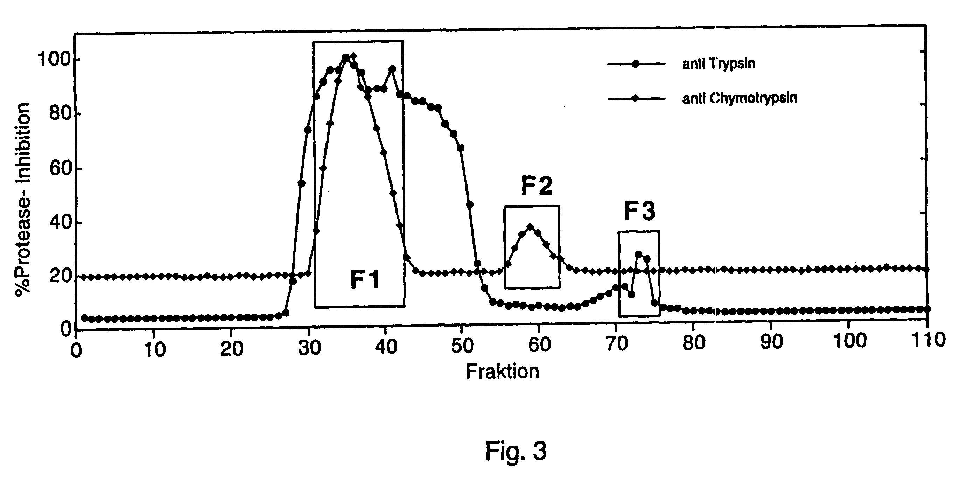

Fig. 3 zeigt das Ergebnis der Protease-Inhibition der einzelnen Säulenfraktionen, wobei die punktförmigen Symbole die Trypsin-inhibierende Aktivität (anti Trypsin) und die rautenförmigen Symbole die Chymotrypsin-inhibierende Aktivität (anti Chymotrypsin) darstellen.

Es zeigten sich insgesamt drei Peaks: ein Peak (F1) bei den Fraktionen 30-50, in dem sowohl Trypsin-inhibierende Aktivität als auch Chymotrypsin-inhibierende Aktivität nachweisbar war, ein weiterer Peak (F2) bei Fraktion 60, in dem Chymotrypsin-inhibierende Aktivität nachweisbar war, und ein dritter Peak (F3) bei Fraktion 72-73, in dem Chymotrypsin-inhibierende Aktivität nachweisbar war.

Die in dem ersten Peak (F1) enthaltenen Fraktionen beinhalten vorwiegend den ungespaltenen Gesamt-BBI, der sowohl Trypsin als auch Chymotrypsin inhibiert. Die mit F2 und F3 bezeichneten Fraktionen enthalten zwei Spaltprodukte des BBI, einmal das Spaltprodukt mit Chymotrypsin-inhibierender Aktivität (F2) und das Spaltprodukt mit Trypsin-inhibierender Aktivität (F3).

### 2.4 Klonogener Assay

Die drei Fraktionen F1, F2 und F3 wurden in einen klonogenen Assay eingesetzt, wie er unter 1.1 beschrieben wurde. Die Hautfibroblasten wurden vor der Bestrahlung mit einer Einzeldosis von 2 Gy für 16 Stunden mit den Fraktionen F1 bis F3 behandelt und deren klonogenes Überleben im Vergleich zu bestrahlten Kontrollzellen (O) ohne Zusatz eines BBI-Produktes analysiert.

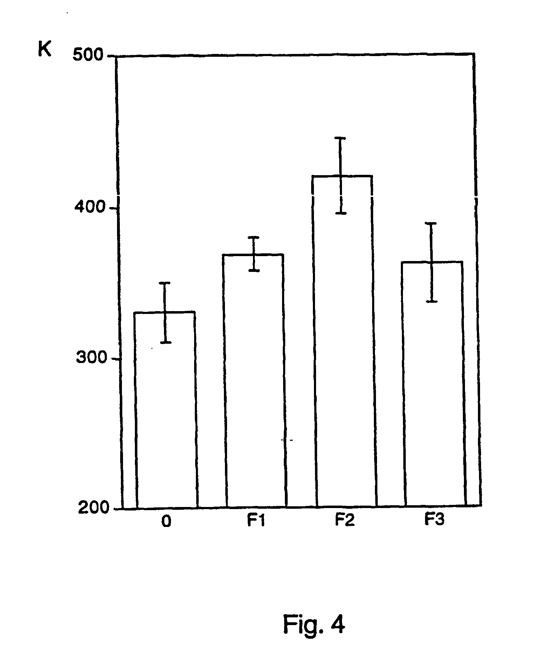

Alle drei Fraktionen zeigten gemäß Fig. 4 eine deutliche Steigerung des klonogenen Überlebens, wobei die Fraktion F2, also das Spaltprodukt mit der Chymotrypsin-inhibierenden Aktivität, sogar einen wesentlich höheren radioprotektiven Effekt als ungespaltener Gesamt-BBI (F1) oder Spaltprodukt mit Trypsin-inhibierender Aktivität (F3) aufwies.

Durch die hier gezeigten Ergebnisse ist nachgewiesen, daß durch chemische und enzymatische Spaltung erzeugte Peptidfragmente des BBI eine ebenso hohe oder sogar wesentlich höhere radioprotektive Wirkung als der Gesamt-BBI entfalten.

### Beispiel 3 Radioprotektive Wirkung der erfindungsgemäßen Nona- und Heptapeptide

In einem weiteren klonogenen Assay (siehe 1.1) wurde die radioprotektive Wirkung von insgesamt vier erfindungsgemäßen BBI-Fragmenten im Vergleich zu Gesamt-BBI untersucht.

In den Test eingesetzt wurde Gesamt-BBI (BBI) sowie vier durch Merrifield-Synthese chemisch synthetisierte Peptide, deren C-terminale Aminosäuren Acetylgruppen und deren N-terminale Aminosäuren Amidgruppen als Schutzgruppen aufwiesen. Die Sequenzen dieser Peptide sind im beigefügten Sequenzprotokoll angegeben.

Das Peptid P1 weist die Sequenz SEQ ID-Nr.: 1 auf, wobei seine terminalen Cysteinreste über eine Disulfidbrücke vernetzt sind, so daß das Peptid P1 eine Ringstruktur aufweist. Das Peptid P1 wird als zyklisches Peptid nur zum Vergleich aufgeführt. Das Peptid P2 hat die Sequenz SEQ ID-Nr.: 3. Es enthält keine Cysteinreste und nimmt daher eine lineare Konformation ein.

Das Peptid P3 entspricht dem Peptid P1, hat also die Sequenz SEQ ID-Nr.: 1, wobei seine terminalen Cysteinreste jedoch nicht über eine Disulfidbrücke vernetzt sind.

Das Peptid P3 hat somit wie P2 eine lineare Struktur.

Das Peptid P4 hat die Sequenz SEQ ID-Nr.: 2 und weist damit gegenüber den Peptiden P1 und P3, die die Sequenz SEQ ID-Nr.: 1 haben, einen Serin → Valin Aminosäureaustausch auf. Seine terminalen Cysteinreste sind nicht über eine Disulfidbrücke vernetzt, so daß auch das Peptid P4 eine lineare Struktur aufweist.

Die Peptide P1, P3 und P4 sind Nonapeptide mit neun Aminosäuren, das Peptid P2 ist dagegen ein Heptapeptid mit nur sieben Aminosäuren.

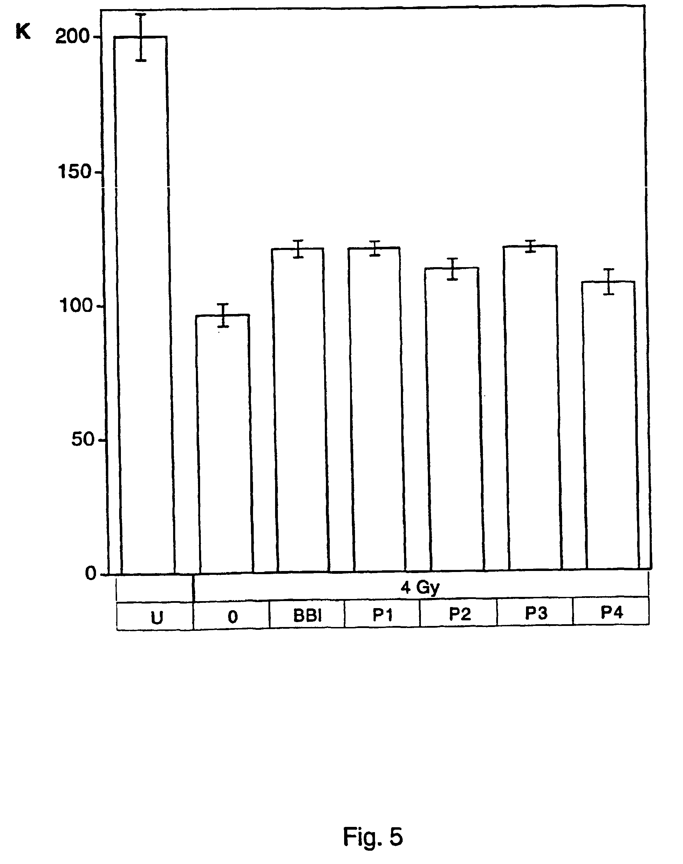

In Fig. 5 ist das Ergebnis des klonogenen Assays gezeigt. Eine Änderung gegenüber dem unter 1.1 beschriebenen Verfahren bestand darin, daß die Bestrahlung der Fibroblasten mit einer Einzeldosis von 4 Gy durchgeführt wurde.

Neben den Ansätzen, bei denen Gesamt-BBI bzw. die Peptide P1 - P4 eingesetzt wurden, wurden ferner zwei Kontrollansätze durchgeführt: einmal ein Ansatz mit unbestrahlten Fibroblasten ohne Zusatz von BBI oder einem Peptid (U), zum anderen ein Ansatz mit bestrahlten Fibroblasten, jedoch ebenfalls ohne Zusatz von BBI oder einem der Peptide (O).

Der Gesamt-BBI wurde mit einer Konzentration von 10 *µ*M, die Peptide P1 - P4 jeweils mit einer Konzentration von 80 *µ*M eingesetzt.

Wie aus Fig. 5 ersichtlich ist, zeigten alle Peptide eine radioprotektive Wirkung, die mit der des Gesamt-BBI vergleichbar ist. Dabei waren die Peptide P1 und P3, also die lineare und die zirkuläre Form der Sequenz SEQ ID-Nr.: 1, gegenüber den Peptiden P2 (Heptapeptid) und P4 (Basenaustausch) in ihrer radioprotektiven Wirkung noch ausgeprägter.

Die erfindungsgemäßen Peptide zeigen somit für menschliche Hautfibroblasten eine Schutzwirkung gegen ionisierende Strahlung, die mit der des acht- bis zehnmal größeren Gesamt-BBI vergleichbar ist.

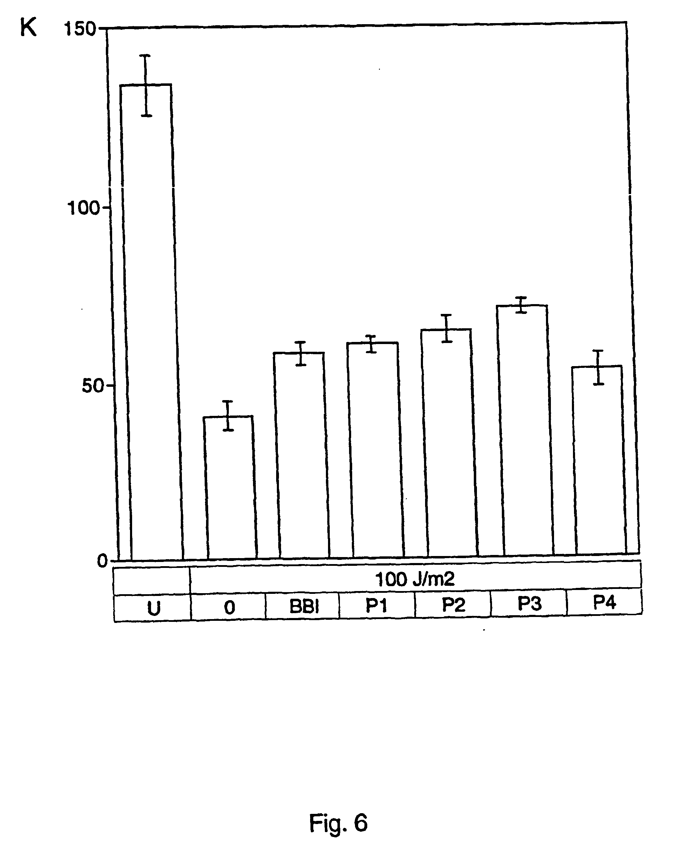

Daß die Peptide nicht nur gegen ionisierende Strahlung, sondern auch gegen UV-Strahlung eine Schutzwirkung ausüben, die ebenso hoch oder höher als die des Gesamt-BBI ist, ist in Fig. 6 gezeigt.

Wieder wurde ein klonogener Assay durchgeführt, in den der Gesamt-BBI oder die vier Peptide P1 bis P4 eingesetzt wurden.

Die Bestrahlung erfolgte hier mit UV-B-Strahlung von 100 Joule/m².

Das klonogene Überleben der menschlichen Hautfibroblasten war unter Zusatz von Gesamt-BBI gegenüber den unbehandelten Zellen um ca. 40 % erhöht. Der Zusatz des Peptids P1 hatte eine ebenso ausgeprägte Wirkung wie der Zusatz von Gesamt-BBI, und der Zusatz der Peptide P2 und P3 zeigte sogar eine ausgeprägtere Wirkung, die um weitere 20 % höher war als die des Gesamt-BBI.

Das Fragment P4, das gegenüber dem Peptid P3 einen Basenaustausch von Serin → Valin aufweist, zeigte gegenüber dem Gesamt-BBI eine geringere Auswirkung auf das klonogene Überleben der Fibroblasten. Im Vergleich zu der Kontrolle (0) steigerte jedoch auch der Zusatz von P4 das klonogene Überleben der Hautfibroblasten signifikant.

Die in Fig. 5 und 6 gezeigten Experimente belegen, daß die erfindungsgemäßen Peptide sowohl gegenüber ionisierender Strahlung als auch gegenüber UV-Strahlung eine radioprotektive Wirkung entfalten, die mit der des Gesamt-BBI vergleichbar, teilweise sogar verbessert ist.

Somit sind die erfindungsgemäßen Peptide ideal als radioprotektive Agenzien einsetzbar.

### Beispiel 4 Protease-inhibierende Aktivität der erfindungsgemäßen Peptide

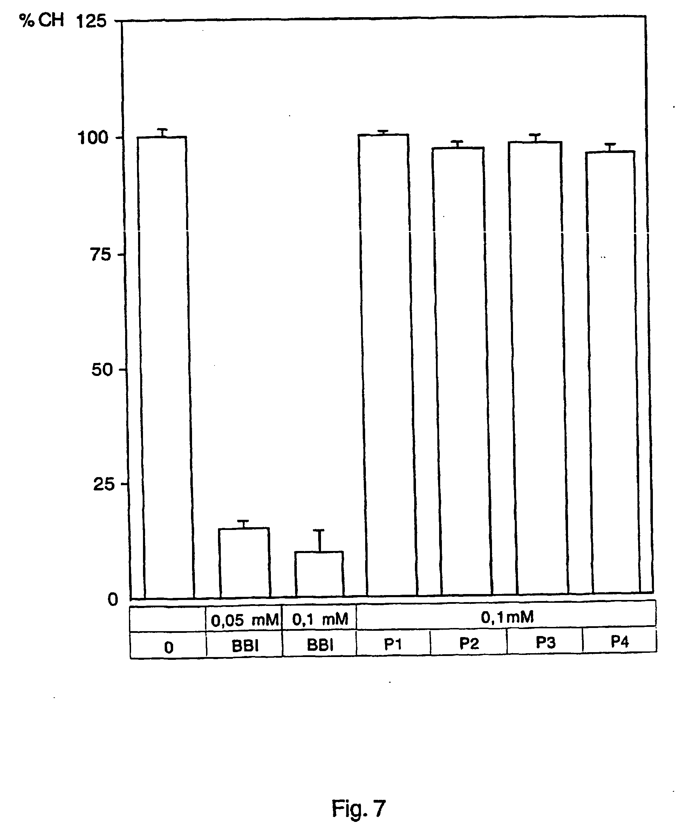

In den Experimenten, deren Ergebnisse in den Fig. 7 und 8 gezeigt sind, wurde die Protease-inhibierende Aktivität des BBI mit der der Peptidfragmente P1 bis P4, die bereits in Beispiel 3 vorgestellt wurden, verglichen.

Der Test der Protease-Inhibition wurde wie unter 1.3 beschrieben durchgeführt. Dabei ist in Fig. 7 die Inhibition von Chymotrypsin und in Fig. 8 die Inhibition von Trypsin gezeigt.

Die mit O bezeichnete Kontrolle zeigt die Chymotrypsin- bzw. Trypsin-Aktivität in Abwesenheit von BBI bzw. einem erfindungsgemäßen Peptid.

Wie aus Fig. 7 ersichtlich ist, inhibiert ein Zusatz von 0,05 mM und 0,1 mM Gesamt-BBI (BBI) die Chymotrypsin-Aktivität um 80 bzw. 90 %. Im Vergleich dazu inhibiert der Zusatz von 0,1 mM des Peptids P1, P2, P3 oder P4 die Chymotrypsin-Aktivität nicht oder nur um wenige Prozent.

Wie aus den in Fig. 8 gezeigten Ergebnissen hervorgeht, gilt das gleiche für die Trypsin-inhibierende Aktivität. Während der Gesamt-BBI in einer Konzentration von 0,05 mM oder 0,1 mM die Aktivität von Trypsin um über 90 % inhibiert, zeigt der Zusatz von 0,1 mM jedes der Peptide P1, P2, P3 oder P4 keine Auswirkung auf die Aktivität von Trypsin.

Dieses Beispiel zeigt, daß keines der erfindungsgemäßen Peptide eine inhibierende Wirkung auf die Proteasen Chymotrypsin oder Trypsin ausübt.

Die erfindungsgemäßen Peptide mit radioprotektiver Wirkung haben daher nicht den unerwünschten Nebeneffekt, gleichzeitig auch die Verdauungsenzyme Chymotrypsin und Trypsin zu blockieren. Wie in Versuchen mit Ratten gezeigt wurde, führt die Blockierung dieser im Pankreas (der Bauchspeicheldrüse) hergestellten Proteasen durch Protease-Inhibitoren zu schweren Schädigungen des Pankreas.

Die erfindungsgemäßen Peptide sind somit dazu geeignet, zum Strahlenschutz des Menschen verwendet zu werden, zumal die Peptide P1 bis P4 aufgrund ihrer geringen Größe schnell diffundieren und sich somit gut verteilen können. Wegen der geringen Größe der Peptide ist auch nicht mit immunologischen Reaktionen zu rechnen.

Eine besonders gute Wirkung entfalten die Peptide P1 bis P4 beim Schutz gegen UV-Strahlung, wie in Ausführungsbeispiel 3 im Zusammenhang mit Fig. 6 gezeigt wurde. Da die nur neun bzw. sieben Aminosäuren umfassenden Peptide sogar in die Haut eindringen können, sind sie zum Schutz der menschlichen Haut gegen erhöhte Sonnenbelastung besonders gut geeignet.
(1) ALLGEMEINE ANGABEN:
   (i) ANMELDER:
      (A) NAME: Eberhard-Karls-Universitaet Tuebingen, Universitaetsklinikum
      (B) STRASSE: Geissweg 3
      (C) ORT: Tuebingen
      (E) LAND: DE
      (F) POSTLEITZAHL: D-72076
   (ii) BEZEICHNUNG DER ERFINDUNG: Peptid mit radioprotektiver Wirkung
   (iii) ANZAHL DER SEQUENZEN: 3
   (iv) COMPUTER-LESBARE FASSUNG:
      (A) DATENTRAEGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID Nr: 1:
   (i) SEQUENZKENNZEICHEN:
      (A) LAENGE: 9 Aminosaeuren
      (B) ART: Aminosaeure
      (C) STRANGFORM: Einzelstrang
      (D) TOPOLOGIE: linear oder circulaer
   (ii) ART DES MOLEKUELS: Peptid
   (iii) HYPOTHETISCH: NEIN
   (iv) ANTISENSE: NEIN
   (xi) SEQUENZBESCHREIBUNG: SEQ ID Nr: 1:
   Cys Ala Leu Ser Tyr Pro Ala Gln Cys
   1               5
(2) ANGABEN ZU SEQ ID NO: 2:
   (i) SEQUENZKENNZEICHEN:
      (A) LAENGE: 9 Aminosaeuren
      (B) ART: Aminosaeure
      (C) STRANGFORM: Einzelstrang
      (D) TOPOLOGIE: linear oder circulaer
   (ii) ART DES MOLEKUELS: Peptid
   (iii) HYPOTHETISCH: NEIN
   (iv) ANTISENSE: NEIN
   (xi) SEQUENZBESCHREIBUNG: SEQ ID Nr: 2:
   Cys Ala Leu Val Tyr Pro Ala Gln Cys
   1               5
(2) ANGABEN ZU SEQ ID Nr: 3:
   (i) SEQUENZKENNZEICHEN:
      (A) LAENGE: 7 Aminosaeuren
      (B) ART: Aminosaeure
      (C) STRANGFORM: Einzelstrang
      (D) TOPOLOGIE: linear
   (ii) ART DES MOLEKUELS: Peptid
   (iii) HYPOTHETISCH: NEIN
   (iv) ANTISENSE: NEIN
   (xi) SEQUENZBES CHREIBUNG: SEQ ID Nr: 3:

## Patentansprüche

1. Peptid mit radioprotektiver Wirkung, das aus der Sequenz SEQ ID-Nr.: 2: oder der Sequenz SEQ ID-Nr. 3: besteht.

2. Peptid nach Anspruch 1, **dadurch gekennzeichnet, daß** seine terminalen Cysteinreste kovalent miteinander verbunden sind.

3. Peptid nach Anspruch 1 oder 2, **dadurch gekennzeichnet, daß** zumindest eine seiner Aminosäuren eine Schutzgruppe aufweist.

4. Peptid nach Anspruch 3, **dadurch gekennzeichnet, daß** seine C-terminale Aminosäure eine Acetylgruppe aufweist.

5. Peptid nach Anspruch 3 oder 4, **dadurch gekennzeichnet, daß** seine N-terminale Aminosäure eine Amidgruppe aufweist.

6. Peptid nach einem der Ansprüche 1 bis 5, **dadurch gekennzeichnet, daß** zumindest einige seiner Aminosäurereste in alkylierter Form vorliegen.

7. Verwendung eines oder mehrerer der Peptide nach einem der Ansprüche 1 bis 6 zur Herstellung eines radioprotektiven Mittels.

8. Verwendung eines oder mehrerer Peptide nach einem der Ansprüche 1 bis 6 zur Herstellung eines Arzneimittels zum Schutz gegen ionisierende Strahlung, insbesondere von Normalgewebe bei der Strahlentherapie von Tumorpatienten.

9. Verwendung eines oder mehrerer Peptide nach einem der Ansprüche 1 bis 6 zur Herstellung eines Mittels zum Schutz der Haut gegen UV-Strahlung, insbesondere gegen die UV-Strahlung des Sonnenlichts.

10. Pharmazeutische Zusammensetzung, insbesondere zur intravenösen Verabreichung, **dadurch gekennzeichnet, daß** sie eines oder mehrere der Peptide nach einem der Ansprüche 1 bis 6 in einer radioprotektiv wirksamen Menge enthält.

11. Kosmetische Zusammensetzung zum Auftragen auf die Haut, **dadurch gekennzeichnet, daß** sie eines oder mehrere der Peptide nach einem der Ansprüche 1 bis 6 in einer radioprotektiv wirksamen Menge enthält.

12. Nukleinsäure, die aus einem für ein Peptid nach einem der Ansprüche 1 bis 6 kodierenden Sequenzabschnitt besteht

13. Nukleinsäure nach Anspruch 12, **dadurch gekennzeichnet, daß** sie in einem Expressionsvektor enthalten ist.

14. Verwendung des Peptides, das aus der Sequenz SEQ ID-Nr.: 1: besteht und in linearer Form vorliegt zur Herstellung eines radioprotektiven Mittels.

## Claims

1. A peptide having a radioprotective effect, consisting of the sequence SEQ ID NO. 2: or of the sequence SEQ ID NO. 3:

2. The peptide of claim 1, **characterized in that** its terminal cysteine residues are covalently bound to each other.

3. The peptide of claim 1 or 2, **characterized in that** at least one of its amino acids comprises a protective group.

4. The peptide of claim 3, **characterized in that** its C-terminal amino acid comprises an acetyl group.

5. The peptide of claim 3 or 4, **characterized in that** its N-terminal amino acid comprises an amide group.

6. The peptide of any of claims 1 to 5, **characterized in that** at least some of its amino acid residues are in alcylated form.

7. Use of one or several of the peptides of any of claims 1 to 6, for the production of a radioprotective composition.

8. Use of one or of several peptides of any of the claims 1 to 6 for the production of a pharmaceutical composition for the protection against ionizing radiation, in particular of normal tissue in radiation therapy of tumor patients.

9. Use of one or of several peptides of any of claims 1 to 6 for the production of a composition for protection of the skin against UV radiation, in particular against the UV radiation of sunlight.

10. A pharmaceutical composition, in particular for intravenous administration, **characterized in that** it contains one or several of the peptides of any of claims 1 to 6 in a radioprotectively effective quantity.

11. A cosmetic composition for application onto the skin, **characterized in that** it contains one or several of the peptides of any of claims 1 to 6 in a radioprotectively effective quantity.

12. A nucleic acid consisting of a sequence segment encoding for a peptide of any of claims 1 to 6.

13. The nucleic acid of claim 12, **characterized in that** it is contained in an expression vector.

14. Use of the peptide consisting of the sequence SEQ ID NO. 1: and that is present in linear form for the production of a radioprotective composition.

## Revendications

1. Peptide à effet radioprotecteur constitué de la séquence SEQ ID N° 2 : ou de la séquence SEQ ID N° 3 :

2. Peptide selon la revendication 1, **caractérisé en ce que** ses résidus cystéines terminaux sont liés entre eux de manière covalente.

3. Peptide selon la revendication 1 ou 2, **caractérisé en ce qu'**au moins un de ses acides aminés présente un groupe protecteur.

4. Peptide selon la revendication 3, **caractérisé en ce que** son acide aminé C-terminal présente un groupe acétyle.

5. Peptide selon la revendication 3 ou 4, **caractérisé en ce que** son acide aminé N-terminal présente un groupe amide.

6. Peptide selon l'une des revendications 1 à 5, **caractérisé en ce qu'**au moins quelques uns de ses résidus d'acides aminés sont présents sous forme alkylée.

7. Utilisation d'un ou plusieurs des peptides selon l'une des revendications 1 à 6 pour fabriquer un agent radioprotecteur.

8. Utilisation d'un ou plusieurs peptides selon l'une des revendications 1 à 6 pour fabriquer un médicament pour protéger contre l'irradiation ionisante, en particulier des tissus normaux dans le cadre d'une radiothérapie appliquée à des patients présentant une tumeur.

9. Utilisation d'un ou plusieurs peptides selon l'une des revendications 1 à 6 pour fabriquer un agent de protection de la peau contre les rayons UV, en particulier contre les rayons UV de la lumière du soleil.

10. Composition pharmaceutique, en particulier pour l'administration intraveineuse, **caractérisée en ce qu'**elle contient un ou plusieurs des peptides selon l'une des revendications 1 à 6 en une quantité radioprotectrice efficace.

11. Composition cosmétique à appliquer sur la peau, **caractérisée en ce qu'**elle contient un ou plusieurs des peptides selon les revendications 1 à 6 en une quantité radioprotectrice efficace.

12. Acide nucléique constitué d'un segment de séquence codant un peptide selon l'une des revendications 1 à 6.

13. Acide nucléique selon la revendication 12, **caractérisé en ce qu'**il est contenu dans un vecteur d'expression.

14. Utilisation du peptide constitué de la séquence SEQ ID N° 1 : et existant sous forme linéaire pour la fabrication d'un agent radioprotecteur.
